# EUROPÄISCHE PATENTSCHRIFT

(11) **EP 0 775 125 B1**
(45) Veröffentlichungstag und Bekanntmachung des Hinweises auf die Patenterteilung: **27.12.2000**
(21) Anmeldenummer: 95929016.4
(22) Anmeldetag: 27.07.1995
(51) Int. Cl.: C07D 275/06, A01N 43/80

(54) **SACCHARINDERIVATE UND IHRE ANWENDUNG ALS HERBIZIDE**
SACCHARINE DERIVATIVES AND THEIR USE AS HERBICIDES
DERIVES SACCHARINE ET LEUR UTILISATION COMME HERBICIDES

(30) Priorität: 08.08.1994 DE 4428000
(43) Veröffentlichungstag der Anmeldung: 28.05.1997
(73) Patentinhaber: BASF AKTIENGESELLSCHAFT, 67056 Ludwigshafen (DE)
(72) Erfinder: PLATH, Peter, D-67227 Frankenthal (DE); VON DEYN, Wolfgang, D-67434 Neustadt (DE); ENGEL, Stefan, D-55286 Wörrstadt (DE); KARDORFF, Uwe, D-68159 Mannheim (DE); NÜBLING, Christoph, D-67454 Ha loch (DE); KÖNIG, Hartmann, D-69115 Heidelberg (DE); RANG, Harald, D-67122 Altrip (DE); GERBER, Matthias, D-67117 Limburgerhof (DE); WALTER, Helmut, D-67283 Obrigheim (DE); WESTPHALEN, Karl-Otto, D-67346 Speyer (DE)
(86) Internationale Anmeldenummer: EP9502977
(87) Internationale Veröffentlichungsnummer: WO9605183

(56) Entgegenhaltungen:
- EP-A- 0 496 630
- EP-A- 0 496 631
- EP-A- 0 506 373
- FR-A- 2 096 157
- FR-A- 2 525 593

## Beschreibung

Gegenstand der vorliegenden Anmeldung sind Saccharinderivate der Formel I 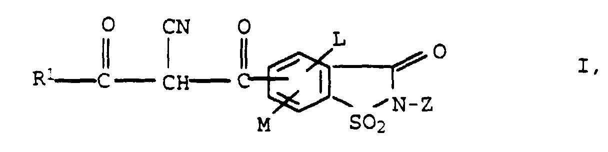 in der die Substituenten folgende Bedeutung haben:
- L,M: Wasserstoff, C₁-C₄-Alkyl, C₁-C₄-Alkoxy, C₁-C₄-Alkylthio,Chlor, Cyano, Methylsulfonyl,Nitro oder Trifluormethyl;
- Z: Wasserstoff, C₁-C₄-Alkyl, C₃-C₈-Cycloalkyl, C₃-C₆-Alkenyl, C₃-C₅-Alkinyl, C₁-C₄-Acyl, Benzyl oder Phenyl, wobei die Phenylringe gegebenenfalls durch Halogen oder C₁-C₄-Alkyl substituiert sind;
- R¹: Cyclopropyl, 1-Methylcyclopropyl , 1-Methylthiocyclopropyl oder tert.-Butyl bedeutet;
sowie landwirtschaftlich übliche Salze der Verbindungen I.

Gegenstand der Erfindung sind ferner herbizide Mittel, enthaltend die Verbindungen I sowie Verfahren zur Bekämpfung unerwünschten Pflanzenwuchses mit den Saccharinderivaten I.

Saccharinderivate mit herbizider Wirkung sind dem Stand der Technik nicht zu entnehmen. Dagegen ist das unsubstituierte Saccharin (o-Sulfobenzoesäureimid) seit langem als synthetischer Süßstoff bekannt. Als Süßstoff bekannt ist ferner das 4-Hydroxy-saccharin (DE-OS 36 07 343). Bekannt ist auch die Verwendung von Saccharinderivaten bei der Schädlingsbekämpfung, z.B. JP-Publikation 72/00419, 73/35457 (Fungizide) und in der Pharmazie, z.B. EP-A 594 257 und darin weiter genannte Patente.

Heterocyclische Verbindungen mit einem eine Sulfonamidgruppe enthaltenden Ring sind als Herbizide bekannt geworden, als typischer Vertreter ist hier das Bentazon 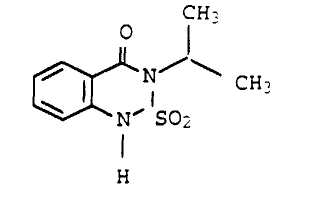 zu nennen.

Der Erfindung lag nun die Aufgabe zugrunde, neue Herbizide mit einer bislang für diese Indikation unbekannten Grundstruktur zur Verfügung zu stellen. Demgemäß wurden die eingangs definierten Verbindungen I gefunden.

Verbindungen der Formel I erhält man dadurch, daß man das Magnesiumenolat A2 eines Cyanoketons der Formel A1 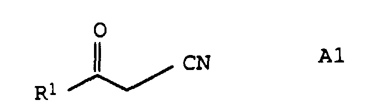 mit einem Saccharincarbonsäurechlorid der Formel III zu der enolisierten Verbindung I.1 acyliert.

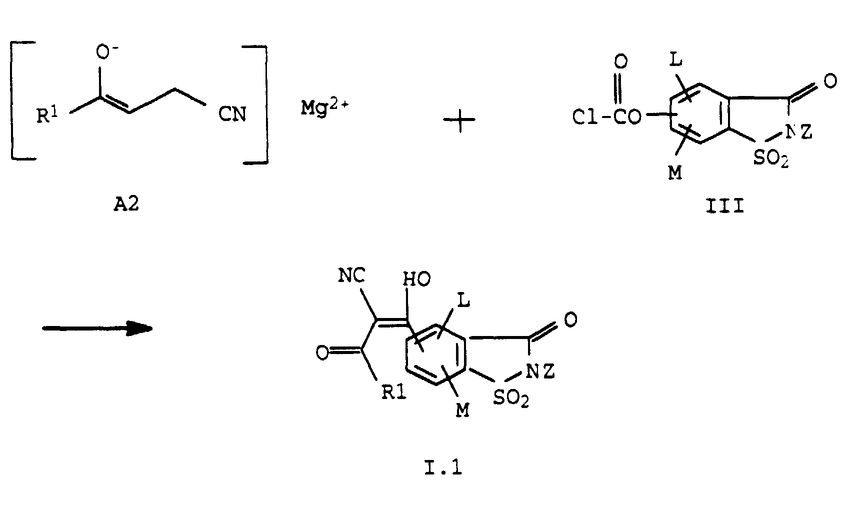

In den oben genannten Formeln haben L und M die eingangs angegebene Bedeutung und Z steht für Wasserstoff, C₁-C₄-Alkyl, C₃-C₈-Cycloalkyl, C₃-C₆-Alkenyl, C₃-C₅-Alkinyl, C₁-C₄-Acyl oder für gegebenenfalls durch Halogen oder C₁-C₄-Alkyl substituiertes Benzyl oder Phenyl.

Der erste Schritt der Reaktionsabfolge erfolgt in der Weise, daß man eine methanolische Lösung eines Cyanoketons der Formel A1 mit Magnesium und einer geringen Menge CCl₄ versetzt und dieses zum Magnesiumenolat abreagieren läßt. Analoge C-Acylierungen von Cyanoketonen sind z. B. in den EP-A 496 630 und EP-A 496 631 beschrieben.

Nach Entfernen des Methanols wird das erhaltene Magnesiumenolat der Formel A2 in Toluol oder Acetonitril gelöst, wobei Acetonitril bevorzugt wird, und dann die zu A1 äquivalente Menge des Säurechlorids der Formel III als Acetonitrillösung zugetropft. Nach 1 - 16 Stunden Rühren bei 25 °C bis 50 °C ist die Umsetzung abgeschlossen. Zur Aufarbeitung wird im Vakuum eingeengt, der verbleibende Rückstand in einem Lösungsmittel wie Essigsäureethylester oder Methylenchlorid aufgenommen und mit 10 proz. HCl gewaschen, um das Magnesiumsalz zu zerlegen. Nach Waschen der organischen Phase mit Wasser wird mit einem Trockenmittel wie Natriumsulfat getrocknet und eingeengt. Aus dem verbleibenden Rückstand wird das Produkt durch Anreiben mit einem Kohlenwasserstoff wie Petrolether, Cyclohexan oder n-Pentan ausgefällt.

Die als Ausgangsstoffe verwendeten Cyanoketone der Formel A1 sind allgemein bekannte Verbindungen. Sie können beispielsweise durch Umsetzung von Cyanessigsäure mit Butyllithium und anschließend mit einem Säurechlorid R¹-COCl erhalten werden.

Die Säurechloride R¹-COCl werden in an sich bekannter Weise aus den entsprechenden Carbonsäuren R¹-COOH durch Umsetzung mit Thionylchlorid erhalten. Die Carbonsäuren R¹-COOH sind literaturbekannte Verbindungen: Pivalinsäure und Cyclopropancarbonsäure sind käufliche Verbindungen. 1-Methylcyclopropancarbonsäure wird entweder durch Verseifung aus dem käuflichen Ethylester erhalten oder in bekannter Weise durch Methylierung des Lithium-α-lithiocylopropancarboxylates [Warner + Le, JOC 47, 893 (1982)] erhalten. 1-Methylthiocyclopropancarbonsäure wird in bekannter Weise aus dem 1-Methyl-thiocyclopropancarbonsäurenitril erhalten, das seinerseits aus Methylmercaptoacetonitril und Ethylendibromid in Gegenwart von Natriumamid hergestellt werden kann [DE-OS 21 20 908 = CA 76: 72099].

Die Ausgangsstoffe der Formel III 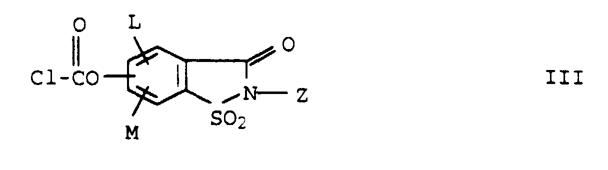 werden in an sich bekannter Weise durch Umsetzung der Saccharincarbonsäuren II 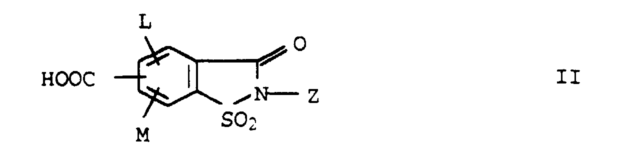 mit Thionylchlorid hergestellt.

Saccharincarbonsäuren II sind zum Teil bekannt (4-COOH: Zincke, Liebigs Ann. 427, 231 (1922), 5-COOH: Jacobsen, Chem. Ber. 13, 1554 (1880), 6-COOH: Weber, Chem. Ber. 25, 1740 (1892)). In der DE-OS 36 07 343 ist ferner die Herstellung der 4-Chlor-saccharin-5-carbonsäure beschrieben.

Saccharincarbonsäuren können auch erhalten werden, indem man entsprechende brom- oder iodsubstituierte Saccharinderivate der Formel A3 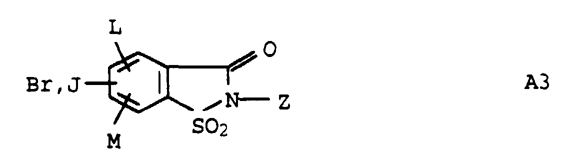 in der L, M und Z die obengenannte Bedeutung haben, oder im Falle von Z ≠ H Verbindungen der Formel A4 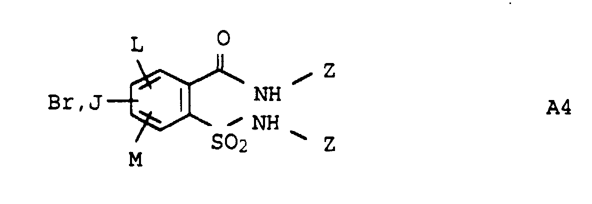 in Gegenwart eines Palladium-, Nickel-, Cobalt- oder Rhodium-Übergangsmetallkatalysators und einer Base mit Kohlenmonoxid und Wasser bzw. einem C₁-C₆-Alkohol unter erhöhtem Druck umsetzt.

Wenn beispielsweise L für Methyl und M und Z für Wasserstoff stehen, läßt sich die Reaktionsfolge wie folgt darstellen:

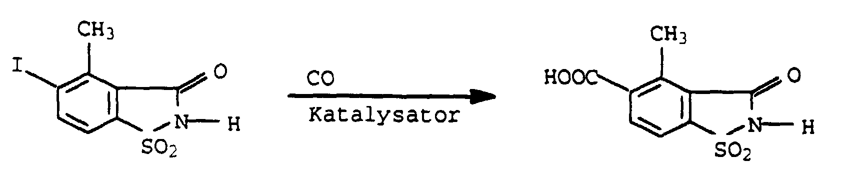

Die Katalysatoren Nickel, Cobalt, Rhodium und insbesondere Palladium können metallisch oder in Form üblicher Salze wie in Form von Halogenverbindungen, z.B. PdCl₂, RhCl₃ · H₂O, z.B. Pd(OAc)₂, Acetaten, Cyaniden usw. in den bekannten Wertigkeitsstufen vorliegen. Ferner können Metallkomplexe mit tertiären Phosphinen, Metallalkylcarbonyle, Metallcarbonyle, z.B. Co₂(CO)₈, Ni(CO)₄, Metallcarbonyl-Komplexe mit tert.-Phosphinen, z.B. (PPh₃)₂Ni(CO)₂ oder mit tertiären Phosphinen komplexierte Übergangsmetallsalze vorliegen. Die letztgenannte Ausführungsform ist insbesondere im Fall von Palladium als Katalysator bevorzugt. Dabei ist die Art der Phosphinliganden breit variabel. Beispielsweise lassen sie sich durch folgende Formeln wiedergeben: 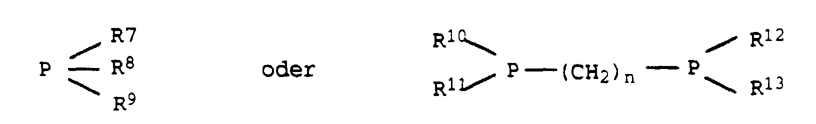 wobei n die Zahlen 1, 2, 3 oder 4 bedeutet und die Reste R⁷ bis R¹³ für niedermolekulares Alkyl, z.B. C₁-C₆-Alkyl, Aryl, C₁-C₄-Alkylaryl, z.B. Benzyl, Phenethyl oder Aryloxy stehen. Aryl ist z.B. Naphthyl, Anthryl und vorzugsweise gegebenenfalls substituiertes Phenyl, wobei man hinsichtlich der Substituenten nur auf deren Inertheit gegenüber der Carboxylierungsreaktion zu achten hat, ansonsten können sie breit variiert werden und umfassen alle inerten C-organischen Reste wie C₁-C₆-Alkylreste, z.B. Methyl, Carboxylreste wie COOH, COOM (M ist z.B. ein Alkali-, Erdalkalimetall oder Ammoniumsalz), oder C-organische Reste über Sauerstoff gebunden wie C₁-C₆-Alkoxyreste.

Die Herstellung der Phosphinkomplexe kann in an sich bekannter Weise z.B. wie in den eingangs genannten Dokumenten, beschrieben hergestellt werden. Beispielsweise geht man von üblichen kommerziell erwerblichen Metallsalzen wie PdCl₂ oder Pd(OCOCH₃)₂ aus und fügt das Phosphin z.B. P(C₆H₅)₃, P(n-C₄H₉)₃, PCH₃(C₆H₅)₂, 1,2-Bis(diphenylphosphino)ethan hinzu.

Die Menge an Phosphin, bezogen auf das Übergangsmetall, beträgt üblicherweise 0 bis 20, insbesondere 0,1 bis 10 Molequivalente, besonders bevorzugt 1 bis 5 Molequivalente.

Die Menge an Übergangsmetall ist nicht kritisch. Natürlich wird man aus Kostengründen eher geringe Menge, z.B. von 0,1 bis 10 Mol.-%, insbesondere 1 bis 5 Mol.-%, bezogen auf den Ausgangsstoff A3 bzw. A4 verwenden.

Zur Herstellung der Saccharincarbonsäuren führt man die Umsetzung mit Kohlenmonoxid und mindestens äquimolaren Mengen an Wasser, bezogen auf die Ausgangsstoffe A3 bzw. A4 durch. Der Reaktionspartner Wasser bzw. C₁-C₆-Alkyl-OH kann gleichzeitig auch als Lösungsmittel dienen, d.h. die maximale Menge ist nicht kritisch.

Es kann aber auch je nach Art der Ausgangsstoffe und der verwendeten Katalysatoren von Vorteil sein, anstelle des Reaktionspartners ein anderes inertes Lösungsmittel oder die für die Carboxylierung verwendete Base als Lösungsmittel zu verwenden.

Als inerte Lösungsmittel kommen für Carboxylierungsreaktionen übliche Lösungsmittel wie Kohlenwasserstoffe, z.B. Toluol, Xylol, Hexan, Pentan, Cyclohexan, Ether z.B. Methyl-tert.-butylether, Tetrahydrofuran, Dioxan, Dimethoxyethan, substituierte Amide wie Dimethylformamid, persubstituierte Harnstoffe wie Tetra-C₁-C₄-alkylharnstoffe oder Nitrile wie Benzonitril oder Acetonitril in Betracht.

In einer bevorzugten Ausführungsform des Verfahrens verwendet man einen der Reaktionspartner, insbesondere die Base im Überschuß, so daß kein zusätzliches Lösungsmittel erforderlich ist.

Für das Verfahren geeignete Basen sind alle inerten Basen, die den bei der Umsetzung freiwerdenden Jodwasserstoff bzw. Bromwasserstoff zu binden vermögen. Beispielsweise sind hier tertiäre Amine wie Triethylamin, cyclische Amine wie N-Methylpiperidin oder N,N'-Dimethylpiperazin, Pyridin, Alkali- oder Erdalkalimetallhydroxide, -carbonate, oder -hydrogencarbonate oder tetraalkylsubstituierte Harnstoffderivate wie Tetra-C₁-C₄-alkylharnstoff, z.B. Tetramethylharnstoff zu nennen.

Die Menge an Base ist nicht kritisch, üblicherweise werden 1 bis 10, insbesondere 1 bis 5 Mol verwendet. Bei gleichzeitiger Verwendung der Base als Lösungsmittel, wird die Menge in der Regel so bemessen, daß die Reaktionspartner gelöst sind, wobei man aus Praktikabilitätsgründen unnötig hohe Überschüsse vermeidet, um Kosten zu sparen, kleine Reaktionsgefäße einsetzen zu können und den Reaktionspartnern maximalen Kontakt zu gewährleisten.

Während der Umsetzung wird der Kohlenmonoxiddruck so eingestellt, daß immer ein Überschuß an CO, bezogen auf A3 bzw. A4 vorliegt. Vorzugsweise liegt der Kohlenmonoxiddruck bei Raumtemperatur bei 1 bis 250 bar, insbesondere 5 bis 150 bar CO. Bei anderen Temperaturen liegt der Druck entsprechend höher bzw. tiefer.

Die Carbonylierung wird in der Regel bei Temperaturen von 20 bis 250°C, insbesondere bei 30 bis 150°C kontinuierlich oder diskontinuierlich durchgeführt. Bei diskontinuierlichem Betrieb wird zweckmäßigerweise zur Aufrechterhaltung eines konstanten Druckes kontinuierlich Kohlenmonoxid auf das Umsetzungsgemisch aufgepreßt.

Aus dem anfallenden Reaktionsgemisch können die Produkte in üblicher Weise, z.B. durch Destillation isoliert werden.

Die für die Umsetzung benötigten Ausgangsstoffe A3 bzw. A4 sind bekannt oder können in an sich bekannter Weise hergestellt werden. Sie können entweder durch Permanganatoxidation von iodsubstituierten 2-Methylbenzolsulfonamiden oder durch Sandmeyer-Reaktion aus Aminosacchariden erhalten werden. Aminosaccharine werden nach bekannten Methoden durch Reduktion von Nitrosacchariden erhalten, die ihrerseits entweder bekannt sind (Kastle, Amer. Chem. Journal 11, 184 (1889) oder DRP 551423 (1930) oder in literaturbekannter Weise aus geeigneten Nitrobenzolderivaten (Liebigs Ann. 669, 85 (1963)) oder Benzolsulfonamiden synthetisiert werden.

Darüber hinaus können sie analog den Herstellvorschriften aus den Beispielen 1 bis 12 erhalten werden.

Im Hinblick auf die bestimmungsgemäße Verwendung sind Saccharinderivate der Formel I bevorzugt, in der die Reste L bzw. M für Wasserstoff, Methyl, Methoxy, Methylthio, Chlor, Cyano, Methylsulfonyl, Nitro oder Trifluormethyl stehen.

In Formel I bedeutet R¹ besonders bevorzugt Cyclopropyl.

Der Rest Z steht besonders bevorzugt für eine der genannten C-organischen Reste, insbesondere für Methyl, Ethyl, Acetyl, Phenyl oder Propargyl. Insbesondere bevorzugte Wirkstoffe sind der Tabelle 1 zu entnehmen.

Die in Tabelle 1 für einen Substituenten genannten Gruppen stellen außerdem für sich betrachtet - unabhängig von der speziellen Kombination mit anderen Substituenten, in der sie genannt sind - eine besonders bevorzugte Definition des betreffenden Substituenten dar.

Die Verbindungen I können in Form ihrer landwirtschaftlich brauchbaren Salze vorliegen, wobei es auf die Art des Salzes im allgemeinen nicht ankommt. Üblicherweise werden die Salze von solchen Basen in Betracht kommen, welche die herbizide Wirkung von I nicht negativ beeinträchtigen.

Als basische Salze eignen sich besonders diejenigen der Alkalimetalle, vorzugsweise die Natrium- und Kaliumsalze, die der Erdalkalimetalle, vorzugsweise Calcium-, Magnesium-, und Bariumsalze und die der Übergangsmetalle, vorzugsweise Mangan-, Kupfer-, Zink- und Eisensalze sowie die Ammoniumsalze, die ein bis drei C₁-C₄-Alkyl, Hydroxy-C₁-C₄-alkylsubstituenten und/oder einen Phenyl- oder Benzylsubstituenten tragen können, vorzugsweise Diisopropylammonium-, Tetramethylammonium-, Tetrabutylammonium-, Trimethylbenzylammonium-, und Trimethyl-(2-hydroxyethyl)-ammoniumsalze, die Phosphoniumsalze, die Sulfoniumsalze, vorzugsweise Tri-(C₁-C₄-)alkylsulfoniumsalze, und die Sulfoxoniumsalze, vorzugsweise Tri-(C₁-C₄-)alkylsulfoxoniumsalze.

Die Verbindungen I bzw. die sie enthaltenden herbiziden Mittel sowie deren umweltverträgliche Salze von beispielsweise Alkalimetallen, Erdalkalimetallen oder Ammoniak und Aminen bzw. die sie so enthaltenden herbiziden Mittel können in Kulturen wie Weizen, Reis, Mais, Soja und Baumwolle Unkräuter und Schadgräser sehr gut bekämpfen, ohne die Kulturpflanzen nennenswert zu schädigen. Dieser Effekt tritt vor allem bei niedrigen Aufwandmengen auf.

Unter Berücksichtigung der Vielseitigkeit der Applikationsmethoden können die Verbindungen Ia, Ia' bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber lassen sich die Verbindungen I auch in Kulturen, die durch Züchtung und/oder mittels gentechnischer Methoden gegen die Wirkung von I oder anderen Herbiziden weitgehend resistent gemacht wurden, einsetzen.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline oder deren Derivate, alkylierte Benzole und deren Derivate, Alkohole wie Methanol, Ethanol, Propanol, Butanol und Cyclohexanol, Ketone wie Cyclohexanon oder stark polare Lösungsmittel, z.B. Amine wie N-Methylpyrrolidon, oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.-%, vorzugsweise zwischen 0,5 und 90 Gew.-%, Wirkstoff. Die Wirkstoffe werden dabei in einer Reinheit von 90 % bis 100 %, vorzugsweise 95 % bis 100 % (nach NMR-Spektrum) eingesetzt. Die erfindungsgemäßen Verbindungen I können beispielsweise wie folgt formuliert werden:
- I.: 20 Gewichtsteile der Verbindung 1.03 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
- II.: 20 Gewichtsteile der Verbindung 1.03 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- III.: 20 Gewichtsteile des Wirkstoffs 1.03 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- IV.: 20 Gewichtsteile des Wirkstoffs 1.03 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
- V.: 3 Gewichtsteile des Wirkstoffs 1.03 werden mit 97 Gewichtsteilen feinteiligem Kaolin vermischt. Man erhält auf diese Weise ein Stäubemittel, das 3 Gew.-% des Wirkstoffs enthält.
- VI.: 20 Gewichtsteile des Wirkstoffs 1.03 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Zur Verbreiterung des Wirkungsspektrums und zur Erzielung synergistischer Effekte können die Saccharincarbonsäurederivate I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele:

### 1. 2-Methyl-6-acetaminobenzoesäure

Zu einer Lösung von 24,8 g (0.62 Mol) NaOH in 500 ml Wasser gibt man 90,6 g (0.6 Mol) 6-Methylanthranilsäure und tropft dann 63,4 g (0.62 Mol) Acetanhydrid zu. Nach einer Stunde Nachrühren wird unter Kühlung mit. konz. HCl auf pH 3 angesäuert, der ausfallende Niederschlag wird abgesaugt, mit Wasser gewaschen und bei 50 °C i. Vak. getrocknet. Ausbeute; 107 g (0.55 Mol) = 92 % d.Th., Fp.: 189 - 190 °C

### 2. 2-Methyl-3-nitro-6-acetaminobenzoesäure

Man legt 271 ml 98 proz. Salpetersäure bei - 5°C vor und trägt portionsweise 106 g (0,55 Mol) der unter 1. hergestellten 2-Methyl-6-acetaminobenzoesäure ein. Nach einer Stunde Nachrühren bei 10 °C wird das Reaktionsgemisch in eine Mischung aus 540 g Eis und 270 ml Wasser gegossen. Der ausgefallene Niederschlag wird abgesaugt, mit Wasser gewaschen und bei 50 °C i. Vak. getrocknet.
Ausbeute: 75,6 g (0.317 Mol) = 58 % d. Th., Fp.: 190 - 191 °C
Aus dem Filtrat scheidet sich nach längerem Stehen das in 3-Stellung nitrierte Isomere ab. Ausbeute: 21,3 g (0.089 Mol) = 16 % d. Th., Fp.: 180 - 182 °C

### 3. 2-Methyl-3-nitro-6-aminobenzoesäure

Man legt 450 ml 2-N NaOH vor und fügt 75,6 g (0.317 Mol) 2-Methyl-3-nitro-6-acetaminobenzoesäure zu. Anschließend erwärmt man die Reaktionsmischung auf 95 °C und läßt eine Stunde bei dieser Temperatur rühren. Nach Abkühlung auf 10 °C wird durch Zugabe von 425 ml 2-N HCl angesäuert. Man saugt den ausfallenden Niederschlag ab, wäscht mit Wasser und trocknet bei 50 °C i. Vak. .
Ausbeute: 50,7 g (0.258 Mol) = 82 % d. Th., Fp.: 183 - 184 °C

### 4. 2-Methyl-3-nitro-6-aminobenzoesäuremethylester

Man löst 49,7 g (0.253 Mol) 2-Methyl-3-nitro-6-aminobenzoesäure in 380 ml Aceton und fügt 43 g (0.51 Mol) Natriumhydrogencarbonat zu. Anschließend wird zum Sieden erhitzt, bis die CO₂-Entwicklung abgeschlossen ist. Zu der so erhaltenen Suspension des Natriumsalzes von 2-Methyl-3-nitro-6-aminobenzoesäure tropft man anschließend bei Siedetemperatur des Acetons 35,3 g (0.28 Mol) Dimethylsulfat im Laufe von zwei Stunden zu, refluxiert anschließend noch drei Stunden und läßt dann abkühlen. Nach Eingießen der Reaktionsmischung in 1,8 l Wasser wird mit Methylenchlorid extrahiert. Nach Trocknen der organischen Phase wird eingeengt. Der erhaltene Feststoff ist für die Folgeumsetzung genügend rein (NMR).
Ausbeute: 50 g (0.238 Mol) = 94 % d. Th., Fp.: 92 - 94 °C

### 5. 2-Methoxycarbonyl-3-methyl-4-nitro-benzolsulfonsäurechlorid

Man löst unter Erwärmen 58,5 g (0.278 Mol) 2-Methyl-3-nitro-6-amino-benzoesäure-methylester in 280 ml Eisessig und gießt diese Lösung bei 15 - 20 °C in 85 ml konz. HCl. Dann tropft man bei 5 - 10 °C eine Lösung von 19,3 g (0.28 Mol) Natriumnitrit in 60 ml Wasser zu und läßt 30 min bei 5 °C nachrühren. Anschließend tropft man diese Diazoniumsalz-lösung in eine Lösung von 374 g SO₂ in 750 ml Eisessig, die 14 g CuCl₂ (in 30 ml Wasser gelöst) enthält. Nach Beendigung der Stickstoffentwicklung wird noch 15 min nachgerührt und dann in 1,4 l Eiswasser gegossen. Das Sulfonsäurechlorid wird durch Extraktion mit 1,2 l Methylenchlorid abgetrennt. Nach Trocknen und Einengen der organischen Phase erhält man 73 g (0.25 Mol) (= 90 % d.Th.) eines Öls, das nach NMR (in CDCl₃) reines 2-Methoxycarbonyl-3-methyl-4-nitro-benzolsulfonsäurechlorid ist.

### 6. 4-Methyl-5-nitrosaccharin

Man legt 104 ml 25 proz. Ammoniaklösung vor, fügt 100 ml Wasser zu und tropft dann bei 10 °C eine Lösung von 48,7 g (0.166 Mol) 2-Methoxycarbonyl-3-methyl-4-nitro-benzolsulfonsäurechlorid in 70 ml Tetrahydrofuran zu. Nach drei Stunden Rühren bei 25 °C wird am Rotationsverdampfer eingeengt, um Wasser und THF zu entfernen. Der verbleibende Rückstand wird mit Essigester verrührt, abgesaugt und mit Essigester gewaschen. Nach dem Trocknen i. Vak. erhält man 34 g (0.131 Mol) = 79 % d. Th. eines weißen Feststoffs mit Fp.: 312 °C (Zers.)

### 7. 2,4-Dimethyl-5-nitrosaccharin

Diese Substanz kann durch nachträgliche Methylierung des unter 6. erhaltenen Saccharins mit Dimethylsulfat in Gegenwart von NaOH hergestellt werden.

### 8. 3-Methyl-4-nitro-2-(N'-methyl)carboxamido-N-methylbenzolsulfonamid

Man gießt 50 ml Wasser in 50 ml 40 proz. Methylamin-Lösung und tropft bei 10 °C eine Lösung von 24,3 g (83 mMol) 2-Methoxycarbonyl-3-methyl-4-nitro-benzolsulfonsäurechlorid in 35 ml THF zu. Nach einer Stunde Rühren bei 25 °C werden alle flüchtigen Bestandteile am Rotationsverdampfer abgezogen, der Rückstand wird mit Essigester extrahiert, die organische Phase mit Wasser gewaschen, getrocknet und eingeengt. Der verbleibende Rückstand kristallisiert nach längerem Stehen. Ausbeute: 10,3 g (40 mMol = 48 % d. Th.), Fp.: 125 - 126 °C, nach Umkristallisation aus Essigester Fp.: 144 - 145 °C.
Das Produkt ist nach NMR kein Saccharinderivat sondern ein Carbonsäureamid mit einer zusätzlichen Sulfonamidgruppe.

### 9. 4-Methyl-5-amino-saccharin

Man löst unter Erwärmen auf 45 °C 33,6 g (0.13 Mol) 4-Methyl-5-nitro-saccharin in 1,2 l Wasser und fügt 5 g Pd/C (10 proz. auf Aktivkohle) zu. Anschließend leitet man unter heftigem Rühren Wasserstoffgas ein (drucklose Hydrierung). Im Laufe von 4,5 Stunden werden 9 l H₂ aufgenommen. Nach Abkühlen auf 25 °C wird vom Katalysator abfiltriert, am Rotavapor auf 200 ml Volumen eingeengt und dann auf pH 1 angesäuert. Der ausgefallene Niederschlag wird abgesaugt, mit Wasser gewaschen und bei 50 °C i. Vak. getrocknet. Man erhält 23,4 g (0. 11 mol = 85 % d. Th.) eines weißen Feststoffs mit Fp.: 272 - 273 °C

### 10. 4-Methyl-5-iod-saccharin

Man legt eine Mischung von 205 ml Eisessig, 160 ml Wasser und 40 ml konz. HCl vor und trägt unter Rühren 23,4 g (0.11 Mol) 4-Methyl-5-amino-saccharin bei 15 - 20 °C ein. Zu der entstandenen Suspension tropft man bei 5 - 10 °C 7,9 g (0.115 Mol) Natriumnitrit und läßt 30 min. bei 5 °C nachrühren. Das als Suspension vorliegende Diazoniumsalz wird dann portionsweise in eine auf 50 °C erwärmte Lösung von 19,1g (0.115 Mol) Kaliumiodid in 170 ml Wasser getropft, wobei Stickstoff entsteht. Nach Abkühlen auf Raumtemperatur wird das ausgefallene Produkt durch Absaugen isoliert, mit Wasser gewaschen und bei 50 °C i. Vak. getrocknet. Man erhält 32,5 g (0.1 Mol = 91 % d. Th.) eines Feststoffs mit Fp.: 257 - 258 °C.
Eine Verbrennungsanalyse ergab einen Iodgehalt von 38,5 % (Theorie 39, 3 %).
Das Produkt ist für die Folgeumsetzungen genügend rein.

### 11. 4-Methyl-saccharin-5-carbonsäure

6,4 g (0,002 mol) 4-Methyl-5-iodsaccharin werden in 70 ml Tetramethylharnstoff und 30 ml Wasser 0,7 g gelöst, mit Bis(triphenylphosphin)palladiumchlorid versetzt und das Gemisch in einem 300-ml-Autoklaven auf 100°C erhitzt und 36 h bei einem Druck von 100 bar Kohlenmonoxid gerührt.
Zur Aufarbeitung filtriert man und entfernt Wasser und Tetramethylharnstoff destillativ im Hochvakuum. Der Rückstand wird in Methyltert.-butylether (MTBE) aufgenommen, mit NaHCO₃-Lsg. extrahiert und nach dem Ansäuern mit HCl wieder mit MTBE extrahiert. Nach dem Einengen erhält man 2,8 g 4-Methyl-saccharin-5-carbonsäure (58 % d. Th.).
¹H-NMR (DMSO, 400,1 MHz): 2,85 (3H, s); 8,05 (1H, d); 8,2 (1H, d);
¹³C-NMR (DMSO, 100, 6 MHz): 167,4 (CO); 161,3 (CO); 141,6 (quart. C); 139,7 (quart. C); 138,7 (quart. C); 135,6 (CH); 125,4 (quart. C); 118,5 (CH); 15,4 (CH₃).

### 12. 2,4-Dimethyl-saccharin-5-carbonsäure

7,3 g (0,02 mol) 3-Methyl-4-iod-2-(N'-methyl)carboxamido-N-methylbenzol- sulfonamid werden zusammen mit 0,69 g Bis(triphenylphosphin)palladiumchlorid, 30 ml Wasser und 70 ml Tetramethylharnstoff in einem 300-ml-Autoklaven vorgelegt, das Gemisch auf 100 °C erhitzt und 36 h bei einem Druck von 100 bar Kohlenmonoxid gerührt.
Nach Aufarbeitung wie in Beispiel 12 beschrieben erhält man 4,1 g der Titelverbindung 2,4-Dimethylsaccharin-5-carbonsäure (0,014 mol = 72 % d. Th.).
¹H-NMR (DMSO, 400,1 MHz): 2,9 (3H, s); 3,15 (3H, s); 8,2 (2H, 2d); 14,0 (1H, s)
¹³C-NMR (DMSO, 100,6 MHz): 167,3 (CO); 158,6 (CO); 139,7 (quart. C); 139,1 (quart. C); 138,9 (quart. C); 135,5 (CH); 124,6 (quart. C); 119,0 (CH); 22,9 (CH3); 15,6 (CH₃).

### 13. 4-Amino-3-methyl-2-(N'-methyl)carboxamido-N-methylbenzolsulfonamid

Analog dem bei Punkt 9 beschriebenen Vorgehen wurde das bei Punkt 8 erhaltene 3-Methyl-4-nitro-2-(N'-methyl)carboxamido-N-methyl-benzolsulfonamid drucklos hydriert. Man erhielt in 93 % Ausbeute ein Anilinderivat der Struktur 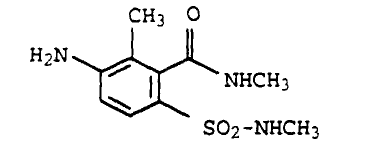 mit Fp.: 217- 218 °C

### 14. 3-Methyl-4-iod-2-(N'-methyl)carboxamido-N-methylbenzolsulfonamid

Nach dem bei Punkt 10 beschriebenen Vorgehen wurde die voranstehende Verbindung diazotiert und durch Umsetzung mit KI zu dem Iodbenzolderivat Struktur umgesetzt. Ausbeute: 95 %, Fp.: 60 - 62 °C

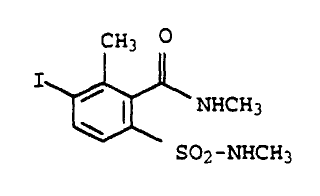

### 15. 2,4-Dimethyl-saccharin-5-carbonsäurechlorid

Man suspendiert 3,8 g (14,9 mMol) der 2,4-Dimethyl-saccharin-5-carbonsäure in 100 ml Toluol, erwärmt auf 80 °C und tropft 3,5 g (29,8 mMol) Thionylchlorid zu. Nach zwei Stunden Refluxieren wird heiß dekantiert und das Reaktionsgemisch eingeengt. Das erhaltene Produkt (3g, 74 % d. Th.) hat Fp.: 149- 150 °C.

### 16. Allgemeine Herstellungsvorschrift der Wirkstoffe der Formel I

Zu einer Lösung von 15 mMol des jeweiligen Cyanoketons der Formel A1 in 50 ml Methanol werden 0,43 g (18 mMol) Magnesiumspäne gegeben und bei 25 °C tropfenweise 1 ml CCl₄ zugetropft. Nach 2 Std. Rühren wird das Methanol i. Vak. abgezogen und der Rückstand in Acetonitril gelöst. Anschließend wird bei 20 - 25 °C eine Lösung von 15 mMol des Saccharincarbonsäurechlorids der Formel III in 20 ml Acetonitril zugetropft und 16 Std. bei 25 °C nachrühren gelassen. Zur Aufarbeitung wird zuerst das Acetonitril i. Vak. abgezogen, der Rückstand in Essigester aufgenommen, von ungelösten Nebenprodukten abfiltriert, die Lösung mit 50 ml 10 proz. HCl ausgeschüttelt, danach zweimal mit Wasser gewaschen, über Natriumsulfat getrocknet und eingeengt. Der ölige Rückstand scheidet beim Anreiben mit Cyclohexan das Produkt der Formel I als kristallinen Feststoff ab.
In gleicher Weise können die folgenden Wirkstoffe in Tabelle 1 erhalten werden:

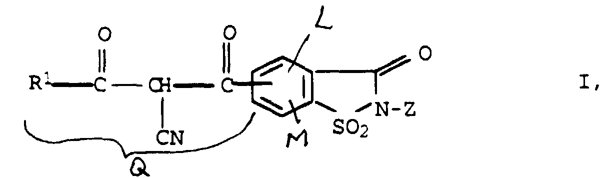

| Nr. | R¹ | Q-Position | L | M | Z | Fp (°C) |
|---|---|---|---|---|---|---|
| 1.01 | cyclo-C₃H₅ | 4 | H | H | H | |
| 1.02 | cyclo-C₃H₅ | 5 | H | H | H | |
| 1.03 | cyclo-C₃H₅ | 6 | H | H | CH₃ | > 225 |
| 1.04 | cyclo-C₃H₅ | 4 | 5-CH₃ | H | CH₃ | |
| 1.05 | cyclo-C₃H₅ | 5 | H | 4-CH₃ | C₂H₅ | |
| 1.06 | cyclo-C₃H₅ | 5 | 4-Cl | H | CH₃ | |
| 1.07 | cyclo-C₃H₅ | 5 | H | 4-Cl | Propargyl | |
| 1.08 | cyclo-C₃H₅ | 6 | H | 4-CH₃ | CH₃ | |
| 1.09 | cyclo-C₃H₅ | 6 | H | 4-Cl | CH₃ | |
| 1.10 | cyclo-C₃H₅ | 6 | 5-Cl | H | C₆H₅ | |
| 1.11 | cyclo-C₃H₅ | 6 | 5-CH₃ | H | C₂H₅ | |
| 1.12 | tert. C₄H₉ | 4 | 5-CH₃ | H | CH₃ | |
| 1.13 | tert. C₄H₉ | 5 | H | H | CH₃ | |
| 1.14 | tert. C₄H₉ | 5 | H | 4-Cl | Propargyl | |
| 1.15 | tert. C₄H₉ | 5 | 4-Cl | H | CH₃ | |
| 1.16 | tert. C₄H₉ | 6 | H | H | H | |
| 1.17 | tert. C₄H₉ | 6 | 5-CH₃ | H | CH₃ | |
| 1.18 | tert. C₄H₉ | 6 | H | 7-Cl | H | |
| 1.19 | tert. C₄H₉ | 6 | H | 7-Cl | CH₃ | |
| 1.20 | tert. C₄H₉ | 6 | 5-CH₃ | H | C₂H₅ | |

### Anwendungsbeispiele

Die herbizide Wirkung der Saccharinderivate der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst darin mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

## Patentansprüche

1. Saccharinderivate der Formel I 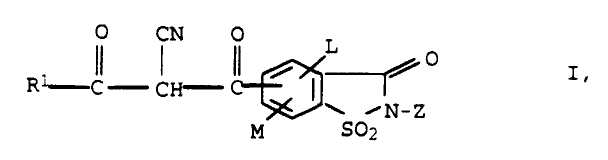 in der die Substituenten folgende Bedeutung haben:
L,M Wasserstoff, C₁-C₄-Alkyl, C₁-C₄-Alkoxy, C₁-C₄-Alkylthio,Chlor, Cyano, Methylsulfonyl,Nitro oder Trifluormethyl;
Z Wasserstoff, C₁-C₄-Alkyl, C₃-C₈-Cycloalkyl, C₃-C₆-Alkenyl, C₃-C₅-Alkinyl, C₁-C₄-Acyl, Benzyl oder Phenyl, wobei die Phenylringe gegebenenfalls durch Halogen oder C₁-C₄-Alkyl substituiert sind;
R¹ Cyclopropyl, 1-Methylcyclopropyl , 1-Methylthiocyclopropyl oder tert.-Butyl bedeutet;
sowie landwirtschaftlich übliche Salze der Verbindungen I.

2. Saccharinderivate der Formel I gemäß Anspruch 1, in der Z Methyl, Ethyl, Propargyl oder Phenyl bedeutet.

3. Saccharinderivate der Formel I gemäß den Ansprüchen 1 und 2, in der die Reste L bzw. M für Wasserstoff, Methyl, Methoxy, Methylthio, Chlor, Cyano, Methylsulfonyl, Nitro oder Trifluormethyl stehen.

4. Herbizides Mittel, enthaltend mindestens ein Saccharinderivat der Formel I gemäß Anspruch 1 und übliche inerte Zusatzstoffe.

5. Verfahren zur Bekämpfung unerwünschten Pflanzenwuchses, dadurch gekennzeichnet, daß man eine herbizid wirksame Menge eines Saccharinderivates der Formel I gemäß Anspruch 1 auf die Pflanzen oder deren Lebensraum einwirken läßt.

6. Verfahren zur Herstellung der Verbindungen der Formel I gemäß Anspruch 1, dadurch gekennzeichnet, daß man Cyanoketone der Formel A1 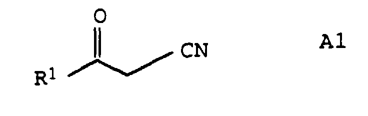 in ihre Magnesiumenolate überführt und diese mit einem Säurechlorid der Formel III 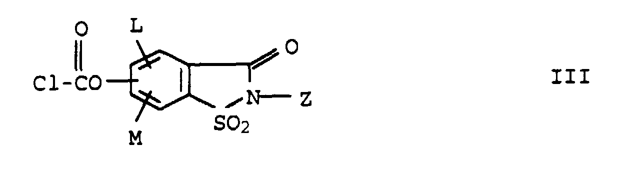 umsetzt.

## Claims

1. A saccharin derivative of the formula I 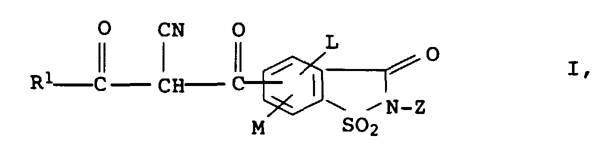 where the substituents have the following meanings:
L and M are hydrogen, C₁-C₄-alkyl, C₁-C₄-alkoxy, C₁-C₄-alkylthio, chlorine, cyano, methylsulfonyl, nitro or trifluoromethyl;
Z is hydrogen, C₁-C₄-alkyl, C₃-C₈-cycloalkyl, C₃-C₆-alkenyl, C₃-C₅-alkynyl, C₁-C₄-acyl, benzyl or phenyl, the phenyl rings being unsubstituted or substituted by halogen or C₁-C₄-alkyl;
R¹ is cyclopropyl, 1-methylcyclopropyl, 1-methylthiocyclopropyl or tert-butyl;
and agriculturally customary salts of the compounds I.

2. A saccharin derivative of the formula I as claimed in claim 1, where Z is methyl, ethyl, propargyl or phenyl.

3. A saccharin derivative of the formula I as claimed in claims 1 and 2, where the radicals L and M are hydrogen, methyl, methoxy, methylthio, chlorine, cyano, methylsulfonyl, nitro or trifluoromethyl.

4. A herbicidal composition, containing at least one saccharin derivative of the formula I as claimed in claim 1 and customary inert additives.

5. A method of controlling undesired plant growth, which comprises allowing a herbicidally active amount of a saccharin derivative of the formula I as claimed in claim 1 to act on the plants or their habitat.

6. A process for preparing the compounds of the formula I as claimed in claim 1, which comprises converting cyanoketones of the formula A1 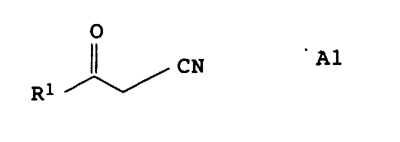 to their magnesium enolates and reacting these with an acid chloride of the formula III

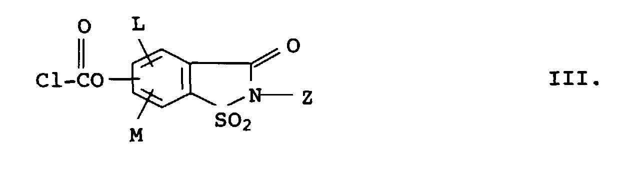

## Revendications

1. Dérivés de la saccharine répondant à la formule I 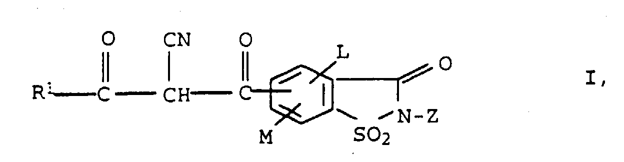 dans laquelle les symboles ont les significations suivantes :
L, M : l'hydrogène, des groupes alkyle en C1-C4, alcoxy en C1-C4, alkylthio en C1-C4, le chlore, des groupes cyano, méthylsulfonyle, nitro ou trifluorométhyle ;
Z : l'hydrogène, un groupe alkyle en C1-C4, cycloalkyle en C3-C8, alcényle en C3-C6, alcynyle en C3-C5, acyle en C1-C4, benzyle ou phényle, les cycles phényle pouvant le cas échéant porter des substituants halogéno ou alkyle en C1-C4 ;
R¹ : un groupe cyclopropyle, 1-méthylcyclopropyle, 1-méthylthiocyclopropyle ou tert-butyle ;
et les sels des composés I usuels pour les applications agricoles.

2. Dérivés de la saccharine de formule I selon la revendication 1, dans laquelle Z représente un groupe méthyle, éthyle, propargyle ou phényle.

3. Dérivés de la saccharine de formule I selon les revendications 1 et 2, dans laquelle les symboles L et M représentent chacun l'hydrogène, un groupe méthyle, méthoxy, méthylthio, le chlore, un groupe cyano, méthylsulfonyle, nitro ou trifluorométhyle.

4. Produit herbicide contenant au moins un dérivé de la saccharine de formule I selon la revendication 1 et des additifs inertes usuels.

5. Procédé pour combattre les croissances de végétaux indésirables, caractérisé par le fait que l'on fait agir une quantité herbicide d'un dérivé de la saccharine de formule I selon la revendication 1 sur les végétaux ou leur habitat.

6. Procédé pour la préparation des composés de formule I selon la revendication 1, caractérisé par le fait que l'on convertit des cyanocétones de formule A1 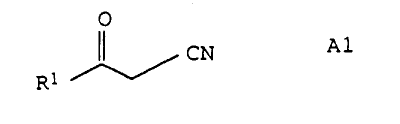 en leurs énolates de magnésium qu'on fait réagir avec un chlorure d'acide de formule III
